# FASCICULE DE BREVET EUROPEEN

(11) **EP 1 405 650 B1**
(45) Date de publication et mention de la délivrance du brevet: **22.03.2006**
(21) Numéro de dépôt: 03292397.1
(22) Date de dépôt: 29.09.2003
(51) Int. Cl.: A61M 35/00, A61B 10/00

(54) **Dispositif de prélèvement**
Entnahmevorrichtung
Sampling device

(30) Priorité: 01.10.2002 FR 0212156
(43) Date de publication de la demande: 07.04.2004
(73) Titulaire: L'OREAL, 75008 Paris (FR)
(72) Inventeur: Kauffmann, Myriam, 75014 Paris (FR); Simon, Pascal, 94400 Vitry Sur Seine (FR)
(74) Mandataire: Tanty, François

(56) Documents cités:
- WO-A-00/09016
- US-A- 5 702 035
- US-A- 5 704 906

## Description

La présente invention concerne les dispositifs de prélèvement, et plus particulièrement mais non exclusivement ceux destinés à prélever à la surface d'un tissu, la peau par exemple, au moins un analyte, par exemple un ion, un composé organique ou de la matière biologique.

L'invention vise notamment à faciliter le prélèvement en vue de l'établissement d'un diagnostic ou d'une évaluation d'un état d'un individu, notamment d'un état de sa peau.

On connaît par US 3 958 571 un applicateur comportant un tube comportant un liquide et un élément d'application à une extrémité du tube. Un tel applicateur est prévu pour appliquer un médicament tel qu'une solution d'iode.

Il existe un besoin pour disposer d'un outil de prélèvement facile à transporter, capable d'être réalisé à un faible coût afin de convenir à un usage unique.

L'invention a pour objet, selon l'un de ses aspects, un dispositif de prélèvement, comportant :
- un tube muni à une extrémité d'un élément de prélèvement,
- un bouchon d'un liquide ou d'une poudre à l'intérieur du tube,
- au moins un produit liquide contenu dans un espace intérieur du tube séparé de l'élément de prélèvement par le bouchon de liquide ou de poudre au moins, ce bouchon étant apte à s'évacuer avec le produit liquide vers l'élément de prélèvement lors de l'utilisation, ce produit liquide pouvant être :
- un liquide de prélèvement propre à faciliter le prélèvement par l'élément de prélèvement d'au moins un analyte, par exemple à imprégner l'élément de prélèvement de manière à rendre ce dernier apte à prélever sur un tissu d'un individu au moins un analyte présent à la surface de ce tissu tout en permettant l'analyse ultérieure de cet analyte, ou
- un réactif propre à produire une réaction observable en présence d'un analyte déterminé recueilli par l'élément de prélèvement.

L'invention permet de disposer d'un moyen relativement simple et peu coûteux pour effectuer un prélèvement sur la peau par exemple, en vue d'effectuer un diagnostic ou une évaluation.

En outre, grâce à l'invention, la quantité de liquide de prélèvement ou de réactif contenu dans le dispositif de prélèvement peut aisément être réduite à la quantité juste nécessaire en choisissant de manière appropriée les dimensions du tube.

Par « analyte », on désigne un composé présent à la surface de la peau, des muqueuses ou des cheveux ou présent dans un fluide corporel tel que l'urine, les larmes, la salive ou la sueur, pouvant réagir avec un réactif spécifique pour être détecté ou quantifié ou en mesurer l'activité. L'analyte peut être présent dans une sécrétion cutanée telle que le sébum. L'analyte peut comporter des micro-organismes et/ou des produits associés à ceux-ci, vivants ou morts, notamment des cellules de peau morte. L'analyte peut encore comporter des lipides ou des enzymes présents par exemple sur la peau ou les muqueuses. L'analyte peut aussi, par exemple, faire partie du groupe constitué par les ions, notamment les ions carbonates, les ions bicarbonates, les ions calcium, les ions chlorure, les ions potassium, les cations métalliques tels que par exemple Cu²⁺, Zn⁺, Fe²⁺ ou Fe³⁺. L'analyte peut encore faire partie du groupe constitué par les composés organiques, notamment les acides aminés libres, les peptides, les protéines ou les hormones.

L'analyte peut encore comporter des résidus de pollution, par exemple d'arsenic, de plomb, de mercure, d'iode ou de césium radioactif.

Par « liquide de prélèvement », on désigne tout liquide permettant de recueillir l'analyte, par exemple en le dissolvant, en le fixant ou en le transformant. Le liquide de prélèvement peut être par exemple choisi dans le groupe constitué par : l'eau, une solution aqueuse, une solution hydro-alcoolique, une solution huileuse, une huile, un solvant organique, le chloroforme, l'acétate d'éthyle, les alcools, les solvants chlorés, l'acétone, les esters courts, les mélanges d'eau et de méthanol, de chloroforme et d'éthanol, cette liste n'étant pas limitative. Il peut en outre comporter un éluant, un fixateur.

Par « réactif », on désigne tout composé en solution ou non, apte à réagir avec un analyte déterminé pour mettre en évidence sa présence, ou quantifier sa concentration ou son activité. Un réactif peut par exemple changer de couleur ou se décolorer lorsqu'il est en contact avec un analyte spécifique. Le réactif peut se présenter sous forme pulvérulente, sous forme de gel, d'hydrogel, de pâte ou de liquide, entre autres.

Lorsque le dispositif comporte un réactif dans le tube, l'élément de prélèvement peut être pré-imprégné par un liquide de prélèvement, le cas échéant.

L'élément de prélèvement peut être poreux, par exemple fibreux, afin par exemple d'être aisément imprégné par le liquide de prélèvement ou par le réactif,

L'élément de prélèvement peut être par exemple choisi dans le groupe constitué par : un embout en coton, un embout en mousse, un embout floqué, une pointe feutre, un embout en céramique ou en un matériau fritté, cette liste n'étant pas limitative.

L'élément de prélèvement peut présenter des propriétés adhésives ou abrasives, afin par exemple de prélever du stratum corneum et/ou des éléments présents à la surface de la peau, d'une muqueuse, des ongles ou des cheveux.

Le bouchon peut comporter, comme indiqué plus haut, un liquide et/ou une poudre.

Lorsque le bouchon comporte un liquide, ce dernier peut être choisi dans le groupe constitué par : les huiles minérales, les produits fluorés, les silicones, cette liste n'étant pas limitative.

Parmi les poudres utilisables pour constituer le bouchon, on peut citer par exemple les poudres comportant des particules organiques ou minérales, pleines ou creuses, par exemple les poudres de microsphères de copolymères telles que l'Expancel® (Nobel Industrie), de Nylon® (notamment l'Orgasol®), des cires, des silices ou des silicones, cette liste n'étant pas limitative.

L'espace intérieur du tube peut être délimité, du côté opposé au bouchon de liquide ou de poudre, par une partie sécable, amovible, perforable ou déformable.

Le dispositif du prélèvement peut être agencé de telle sorte qu'après rupture de l'extrémité sécable, l'utilisateur puisse doser la quantité de liquide qui descend en manipulant le tube comme une pipette dont il obture l'extrémité supérieure avec l'index, le tube pouvant être incliné plus ou moins, le cas échéant.

Dans un exemple de mise en oeuvre de l'invention, le tube peut être rebouché après qu'une fraction seulement du liquide contenu à l'intérieur soit descendue. Ce rebouchage peut s'effectuer par exemple grâce à l'extrémité sécable. Cette dernière peut être configurée par exemple pour pouvoir constituer un bouchon de fermeture, l'extrémité sécable comportant un picot apte à s'engager dans le tube ou sur le tube pour l'obturer.

Le dispositif peut comporter, le cas échéant, un élément de maintien de la partie sécable sur le dispositif de prélèvement, après son sectionnement.

Le volume de produit liquide contenu dans le tube peut être compris par exemple entre 0,01 ml et 5 ml, voire entre 0,05 ml et 5 ml, mieux entre 0,05 ml et 1 ml Le volume de produit liquide peut convenir à un usage unique du dispositif de prélèvement.

Le tube du dispositif de prélèvement peut être réalisé dans une matière transparente, notamment une matière plastique transparente, afin de permettre par exemple à l'utilisateur d'observer le niveau de produit liquide dans le tube ou sa couleur.

Le tube peut comporter une structure multicouche, avec au moins une couche formant barrière vis-à-vis de l'air, par exemple une couche de vernis imperméable à l'air, ou à un solvant ou anti-UV.

L'invention a encore pour objet, selon un autre de ses aspects, un kit de prélèvement et d'analyse comportant :
- au moins un dispositif de prélèvement comportant :
   - un tube,
   - un bouchon d'un liquide ou d'une poudre à l'intérieur du tube,
   - au moins un liquide de prélèvement contenu dans un espace intérieur du tube délimité d'un premier côté par le bouchon de liquide ou de poudre,
   - un élément de prélèvement à une extrémité du tube, l'élément de prélèvement étant séparé du liquide de prélèvement par au moins le bouchon de liquide
   ou de poudre, le liquide de prélèvement étant propre à faciliter le prélèvement d'au moins un analyte, le bouchon de liquide ou de poudre étant apte à s'évacuer avec le liquide de prélèvement vers l'élément de prélèvement lors de l'utilisation,
- un réactif propre à produire, par exemple au sein de l'élément de prélèvement, une réaction observable en présence de l'analyte recueilli par l'élément de prélèvement.

Le kit peut se présenter de diverses manières.

Le kit peut par exemple comporter un boîtier comportant au moins un compartiment dans lequel est logé au moins un dispositif de prélèvement.

En variante, le kit peut comporter au moins un sachet de conditionnement dans lequel est contenu au moins un dispositif de prélèvement.

Le réactif peut être présent par exemple sur des bandelettes contenucs dans le boîtier.

L'invention a encore pour objet, selon un autre de ses aspects, un procédé pour détecter la présence et/ou la concentration d'au moins un analyte, par exemple à la surface d'un tissu, notamment de la peau d'un individu, caractérisé par le fait qu'il comporte les étapes suivantes :
- fournir un dispositif de prélèvement comportant un tube, un bouchon d'un liquide ou d'une poudre à l'intérieur du tube, au moins un liquide de prélèvement contenu dans un espace intérieur du tube délimité d'un premier côté par le bouchon de liquide ou de poudre, un élément de prélèvement à une extrémité du tube, séparé du liquide de prélèvement par le bouchon de liquide ou de poudre,
- ouvrir le tube afin de permettre au liquide de prélèvement de quitter le tube, le bouchon de liquide ou de poudre étant apte à s'évacuer avec le liquide de prélèvement,
- prélever au moins un analyte avec l'élément de prélèvement,
- mettre l'analyte en contact avec un réactif propre à produire une réaction observable en présence de cet analyte ou d'une concentration déterminée de l'analyte.

L'invention a encore pour objet un procédé pour détecter la présence et/ou la concentration d'au moins un analyte, notamment présent à la surface de la peau d'un individu, caractérisé par le fait qu'il comporte les étapes suivantes :
- fournir un dispositif de prélèvement comportant un tube comportant à une extrémité une partie sécable et à l'autre extrémité un élément de prélèvement, un bouchon d'un liquide ou d'une poudre à l'intérieur du tube, au moins un réactif contenu dans un espace intérieur du tube délimité d'un premier côté par le bouchon de liquide ou de poudre,
- sectionner le tube afin de permettre au réactif d'imprégner l'élément de prélèvement, le bouchon de liquide ou de poudre étant apte à s'évacuer avec le réactif vers l'élément de prélèvement, et de mettre en évidence la présence d'un analyte déterminé, éventuellement à une concentration supérieure ou inférieure à un seuil donné.

L'invention a encore pour objet, indépendamment ou en combinaison avec ce qui précède, un procédé d'application d'au moins un produit contenu dans un espace intérieur du tube dans lequel on soumet le tube à une source de chaleur préalablement à l'application du produit. Ce produit peut comporter par exemple au moins un épaississant thermoréversible de telle sorte que le produit se fluidifie lorsque la chaleur augmente. La source de chaleur peut par exemple être la chaleur humaine, ou une source de chaleur externe au corps humain, par exemple. Le fait de fluidifier le produit préalablement à l'application peut permettre d'améliorer sa conservation pendant le stockage dans le tube, notamment de limiter son évaporation. Le fait de fluidifier le produit peut également faciliter son passage à travers un embout d'application, notamment un embout réalisé dans un matériau poreux, par exemple en coton. Le tube peut comporter ou non un bouchon d'un liquide ou d'une poudre disposé d'un côté du produit, ce bouchon de liquide ou de poudre pouvant s'évacuer avec le produit lorsque celui-ci quitte l'espace intérieur du tube lors de l'utilisation.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente de manière schématique un kit de prélèvement et d'analyse,
- les figures 2 à 4 représentent isolément un dispositif de prélèvement,
- les figures 5 à 7 illustrent quelques possibilités d'utilisation du dispositif de prélèvement,
- la figure 8 représente partiellement un autre exemple de kit de prélèvement et d'analyse,
- la figure 9 représente un autre exemple de dispositif de prélèvement,
- les figures 10 à 13 représentent des variantes de réalisation de l'élément de prélèvement,
- les figures 14 à 16 représentent des variantes de réalisation de la partie sécable du dispositif de prélèvement,
- les figures 17 et 18 représentent, de manière schématique, des dispositifs de prélèvement comportant des réactifs bi-phasiques ou multiphasiques,
- la figure 19 représente, de manière schématique et partielle, un autre exemple de dispositif de prélèvement,
- la figure 20 représente, de manière schématique, un récipient destiné à recevoir un ou plusieurs dispositifs de prélèvement, et
- la figure 21 représente, de manière schématique, un support d'un dispositif de prélèvement.

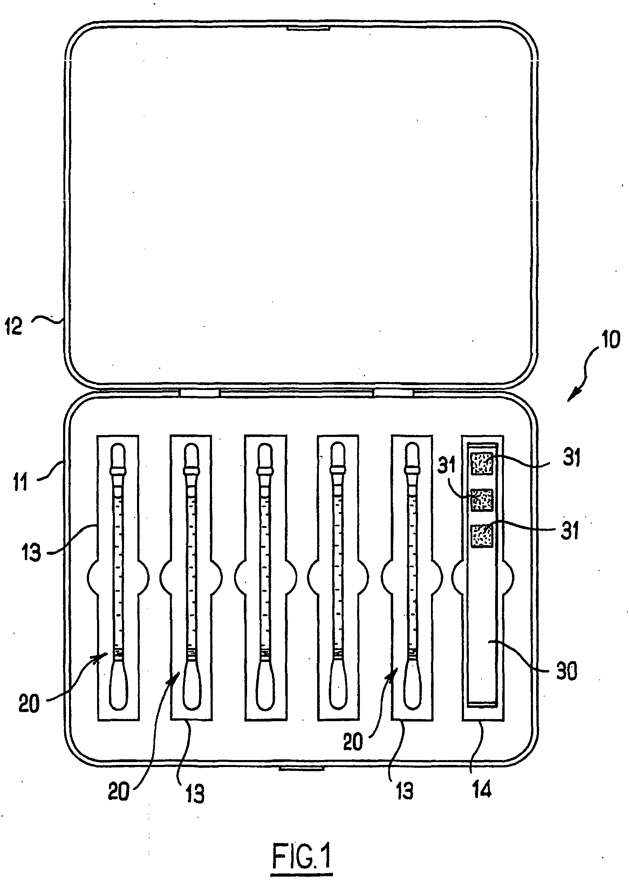

On a représenté à la figure 1, de manière schématique, un kit de prélèvement et d'analyse comportant un boîtier 10 comprenant une partie de base 11 et un couvercle 12 articulé sur la partie de base 11, cette dernière comportant une pluralité de compartiments 13 logeant des dispositifs de prélèvement 20 et un compartiment 14 logeant une pluralité de bandelettes 30 comportant une ou plusieurs zones 31 imprégnées d'au moins un réactif réagissant à un analyte susceptible d'être prélevé au moyen d'un dispositif de prélèvement 20.

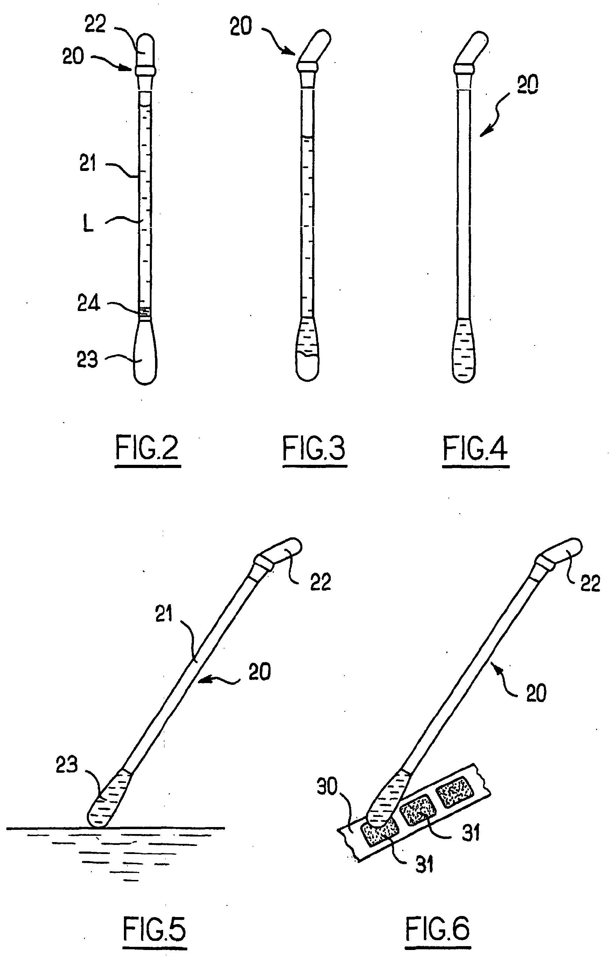

On a représenté isolément, aux figures 2 à 4, un dispositif de prélèvement 20.

Celui-ci peut être, par exemple, un dispositif similaire à celui décrit dans le brevet US 5 702 035 dont le contenu est incorporé ici par référence. Des dispositifs pouvant convenir à la mise en oeuvre de l'invention sont commercialisés par la société californienne Swabplus Inc.

Chaque dispositif 20 comporte un tube 21 contenant par exemple un liquide de prélèvement L, ce tube étant, dans l'ensemble illustré, réalisé par extrusion de matière plastique et pourvu à une extrémité fermée d'une partie sécable 22. Cette dernière est recouverte, dans l'exemple considéré, par un embout de coton. Le tube 21 est ouvert à l'autre extrémité, opposée à la partie sécable 22, étant muni par exemple à cette extrémité d'un élément de prélèvement 23.

Dans l'exemple illustré, l'élément de prélèvement 23 comporte un embout de coton, à la manière d'un coton-tige.

Le liquide de prélèvement L est contenu dans un espace intérieur du tube 21 situé entre la partie sécable 22 et un bouchon 24 présent dans le tube 21, du côté de son extrémité ouverte. Ce bouchon 24 peut être constitué d'un liquide ou d'une poudre.

Le volume de liquide de prélèvement L peut convenir à un usage unique du dispositif de prélèvement, étant déterminé en fonction de la nature du liquide et du prélèvement envisagé, et peut varier par exemple entre 0,01 ml et 5 ml, mieux entre 0,05 ml et 1 ml. Le diamètre extérieur du tube 21 est par exemple inférieur à 6 mm, voire inférieur à 3 mm environ. Le diamètre intérieur du tube 21 peut être compris entre environ 0,5 mm et environ 3 mm, par exemple.

Dans le cas d'un bouchon 24 formé d'un liquide, le liquide du bouchon de liquide 24 peut être constitué par tout liquide inerte compatible avec le conditionnement du liquide présent dans le tube 21, notamment un liquide ne réagissant pas avec le liquide de prélèvement, capable d'être évacué facilement hors du tube 21 au moment de l'utilisation, et par ailleurs physiologiquement acceptable. Le bouchon de liquide 24 permet notamment d'isoler le liquide de prélèvement L de l'air, d'empêcher son évaporation et à des contaminants extérieurs de pénétrer. Le liquide du bouchon de liquide 24 peut être constitué, par exemple, par une huile minérale ou un produit fluoré, entre autres. Dans l'exemple considéré, le bouchon de liquide 24 est formé de silicone.

La quantité de liquide formant le bouchon de liquide 24 est faible par rapport à celle du liquide de prélèvement L.

Lorsque la partie sécable 22 est rompue, l'air peut pénétrer dans le tube 21 du côté opposé à l'extrémité ouverte et le liquide de prélèvement L peut s'écouler par gravité dans le tube 21 et gagner l'élément de prélèvement 23, comme illustré sur les figures 3 et 4, pour être mis au contact d'un tissu d'un individu sur lequel un prélèvement doit être effectué, par exemple sa peau.

Dans l'exemple considéré, l'embout de coton recouvrant la partie sécable 22 permet de maintenir celle-ci solidaire du reste du tube 21, même après sa rupture.

Pour utiliser un dispositif de prélèvement 20, on casse la partie sécable du tube comme illustré à la figure 5, de telle sorte que le liquide de prélèvement L s'écoule dans l'élément de prélèvement 23.

On amène ensuite celui-ci au contact de la peau par exemple, que l'on peut masser légèrement de manière à ce que l'analyte que l'on cherche à mettre en évidence et présent à la surface de la peau se dépose sur l'élément de prélèvement ou diffuse dans le liquide de prélèvement L.

Une fois le prélèvement effectué, on peut amener, comme illustré à la figure 6, l'élément de prélèvement au contact d'une zone 31 imprégnée d'un réactif. Ce dernier peut-être par exemple configuré pour produire une réaction colorée en présence d'un analyte déterminé, recueilli par l'élément de prélèvement 23.

Le liquide de prélèvement L peut par exemple être de l'eau, notamment de l'eau déminéralisée, une solution aqueuse, une solution hydro-alcoolique, une solution alcoolique, une solution huileuse, une huile, un solvant organique, étant choisi en fonction de l'analyte à recueillir, cette liste n'étant pas limitative.

L'analyte dont on cherche à mettre en évidence la présence à la surface de la peau peut par exemple être un ion, notamment un ion carbonate, bicarbonate, calcium ou chlorure.

Une bandelette 30 peut par exemple comporter plusieurs zones 31 comportant des réactifs qui réagissent à des concentrations différentes du même analyte, afin d'effectuer une mesure quantitative de la concentration de l'analyte à la surface de la peau.

Le liquide contenu dans le dispositif de prélèvement peut être un liquide de prélèvement, ainsi que décrit en référence aux figures 1 à 6.

Le tube 21 peut également contenir dans l'espace intérieur situé entre le bouchon 24 et la partie sécable 22, un réactif en solution propre à produire une réaction, notamment colorée, au sein de l'élément de prélèvement 23 en présence d'un analyte déterminé recueilli par l'élément de prélèvement.

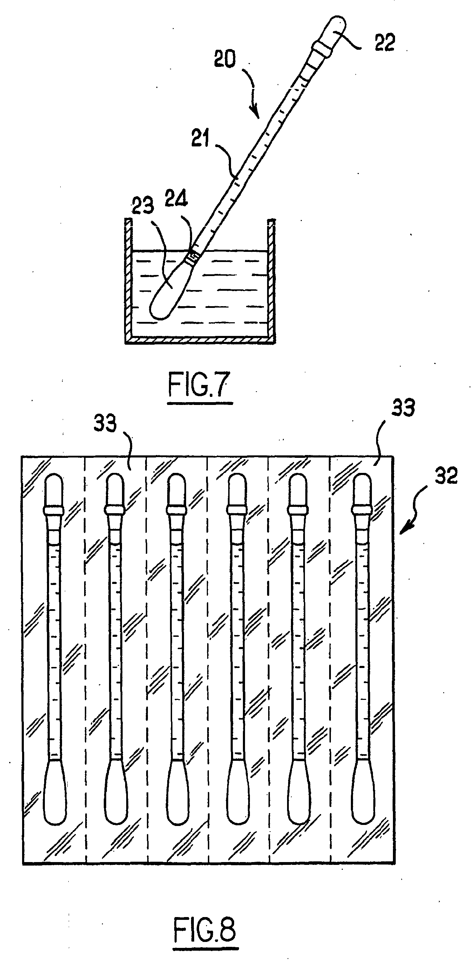

Dans ce cas, on peut commencer par imprégner, comme illustré à la figure 7, l'élément de prélèvement 23 d'un liquide de prélèvement contenu dans un récipient, par exemple de l'eau déminéralisée, puis amener le dispositif 20 au contact de la peau afin d'y prélever éventuellement l'analyte qui pourra réagir ensuite avec le réactif contenu dans le tube 21, une fois la partie sécable du dispositif sectionnée.

On ne sort pas du cadre de la présente invention si l'on prélève un analyte à la surface de la peau sans avoir imprégné au préalable d'un liquide l'élément de prélèvement, notamment si l'analyte en question est contenu dans le sébum ou un fluide corporel.

Le conditionnement des dispositifs de prélèvement peut se présenter sous forme d'un boîtier, comme illustré sur la figure 1, ou sous une autre forme sans que l'on sorte du cadre de la présente invention, par exemple sous forme d'un chapelet 32 de sachets 33, comme illustré à la figure 8.

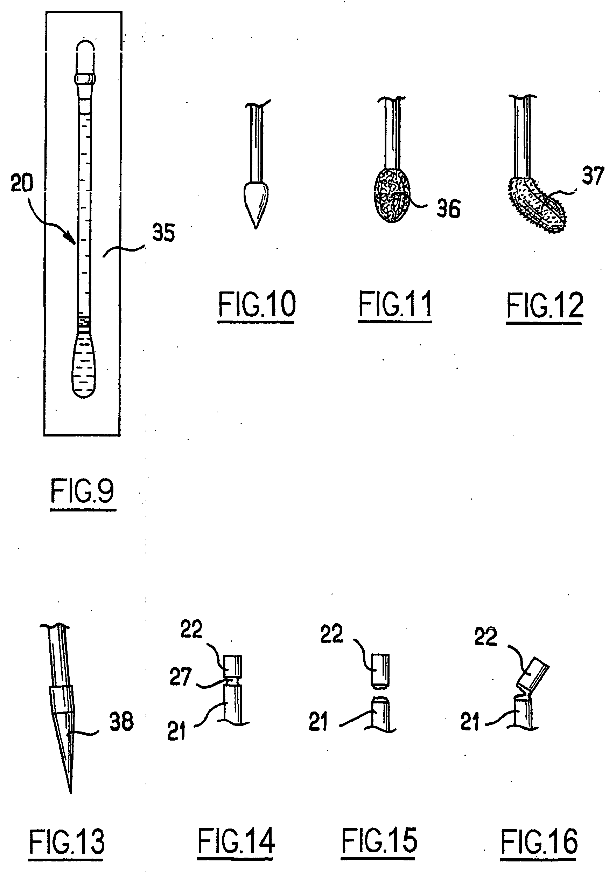

L'élément de prélèvement peut encore être pré-imprégné d'un liquide de prélèvement, comme illustré à la figure 9. Dans ce cas, le liquide contenu à l'intérieur du tube 21 peut contenir un réactif apte à mettre en évidence au moins un analyte susceptible d'être présent à la surface d'un tissu d'un individu.

Le dispositif de prélèvement 20 de la figure 9 peut alors être, ainsi qu'illustré, conditionné dans un sachet de conditionnement hermétique 35.

L'élément de prélèvement peut avoir des formes diverses, et notamment une extrémité arrondie ou effilée, par exemple en forme de flamme comme représenté à la figure 10.

L'élément de prélèvement peut comporter, par exemple, tout matériau poreux, par exemple fibreux, élastiquement compressible ou non.

A titre d'exemple, on a représenté sur la figure 11 un élément de prélèvement qui se présente sous la forme d'un embout en mousse 36.

L'élément de prélèvement peut comporter un flocage 37 à sa surface, comme illustré à la figure 12. On voit également sur cette figure que l'élément de prélèvement peut avoir une forme incurvée, avec une partie s'étendant selon un axe longitudinal non confondu avec l'axe du tube 21.

L'élément de prélèvement peut encore se présenter, par exemple, sous la forme d'un embout 38 ayant une forme effilée, comme illustré à la figure 13. Un tel embout peut être réalisé dans une matière poreuse ou, en variante, dans une matière non poreuse, mais comporter au moins un canal intérieur ou une rainure permettant au liquide contenu dans le tube 21 de s'écouler vers l'extrémité distale.

L'élément de prélèvement peut être réalisé de manière à pouvoir exercer une action abrasive, sur la peau par exemple, en vue de prélever des cellules.

L'élément de prélèvement peut être réalisé par exemple en céramique ou en un matériau fritté.

L'élément de prélèvement peut encore être réalisé en donnant au tube une forme en biseau par exemple, permettant d'effectuer un prélèvement par grattage.

L'élément de prélèvement peut encore présenter des propriétés adhésives.

On a représenté sur les figures 14 à 16 d'autres exemples de réalisation de la partie sécable de l'applicateur.

On voit sur la figure 14 que la partie sécable peut être reliée au reste du tube par une zone de cassure préférentielle 27, matérialisée par exemple par un amincissement de la paroi du tube ou une entaille à ce niveau.

La partie sécable peut être réalisée de différentes autres manières sans que l'on sorte du cadre de la présente invention.

On peut notamment, comme illustré à la figure 15, configurer l'applicateur de telle sorte que la partie sécable 22 puisse être entièrement séparée du tube 21 après avoir exercé manuellement un mouvement de cassure en tenant le tube 21 d'une main et la partie sécable 22 entre deux doigts de l'autre main.

L'applicateur peut encore être configuré de manière à ce que la partie sécable 22 reste solidaire du tube 21 après l'utilisation, comme illustré sur la figure 16, grâce à un pont de matière 50.

L'extrémité du tube peut encore être réalisée autrement, par exemple de l'une des manières représentées sur les figures 3 à 8 du brevet US 3 958 571.

Lorsque l'extrémité sécable peut se détacher entièrement du tube, cela peut faciliter par exemple l'utilisation du dispositif de prélèvement à la manière d'une pipette, l'utilisateur pouvant obturer l'extrémité supérieure de l'élément de prélèvement avec son doigt pour distribuer le produit, par exemple goutte-à-goutte, dans l'élément de prélèvement.

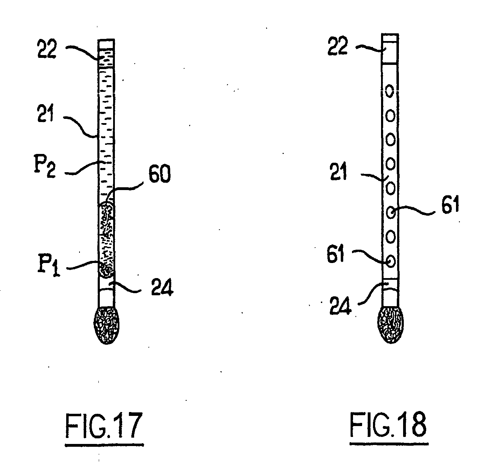

On a représenté à la figure 17 un tube comportant deux réactifs liquides P₁ et P₂ se présentant sous forme de deux phases occupant chacune une portion de la longueur du tube.

Les deux réactifs P₁ et P₂ sont au contact l'un de l'autre par une interface 60.

L'un des réactifs peut aussi se présenter sous la forme d'au moins un globule à l'intérieur de l'autre phase, par exemple sous forme de plusieurs globules 61 comme illustré à la figure 18. Cela peut permettre par exemple de réaliser un dosage en se servant du tube comme d'une pipette ou d'améliorer l'esthétique de l'applicateur.

Plusieurs liquides différents peuvent aussi être dispersés sous la forme de plusieurs globules dans une même phase. Les différents globules peuvent ainsi correspondre à des réactifs ayant des couleurs différentes et/ou contenant des actifs différemment dosés et/ou de natures différentes.

Le produit P₂, lorsque contenu dans le tube, peut encore être solide, étant constitué par exemple par une poudre soluble dans le produit P₁, les produits P₁ et P₂ étant séparés par un bouchon avant l'utilisation. Le volume de produit P₂ peut être suffisamment faible pour que le produit P₂ puisse se dissoudre facilement lors de l'utilisation.

Le nombre de produits présents dans le dispositif de prélèvement peut être supérieur à deux sans que l'on sorte du cadre de la présente invention.

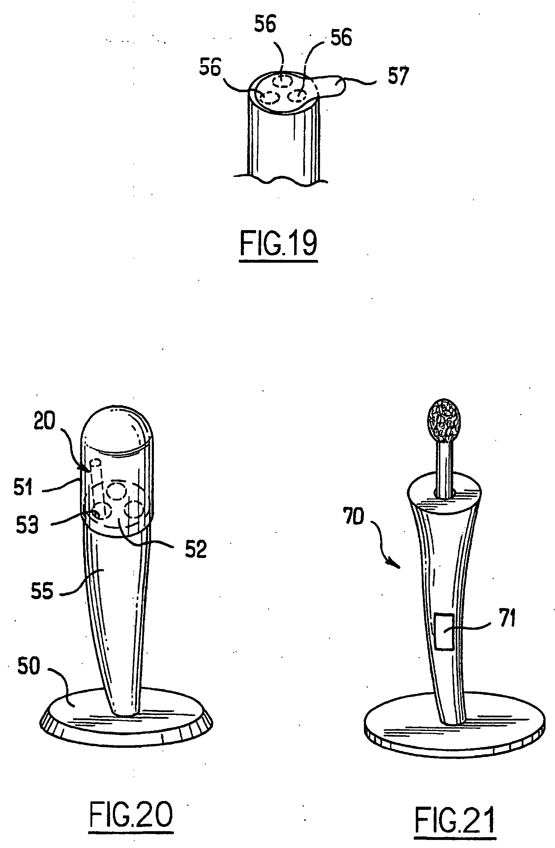

On a représenté à la figure 19 l'extrémité supérieure d'un tube comportant par exemple trois canaux intérieurs 56 contenant par exemple chacun un liquide et un bouchon associé, le tube pouvant être obturé à cette extrémité avant utilisation par un opercule amovible 57, lequel est par exemple collé ou thermosoudé sur le tube.

On a représenté à la figure 20 un récipient pouvant recevoir un dispositif de prélèvement.

Un tel récipient peut par exemple comporter un socle 50 supportant un corps 55 dont l'extrémité supérieure est configurée pour permettre la fixation d'un capuchon de fermeture 51 permettant de fermer de manière sensiblement étanche le récipient, un élément de support 52 étant disposé à l'intérieur du corps, comportant au moins un orifice 53 permettant d'y engager un dispositif de prélèvement 20.

Ainsi, l'utilisateur peut, après un prélèvement, s'il le souhaite, disposer le dispositif de prélèvement à l'intérieur du récipient. La présence du capuchon 51 permet d'éviter que l'élément de prélèvement ne sèche, par exemple.

On peut encore utiliser un dispositif de prélèvement avec un support 70 tel que celui représenté à la figure 21, permettant de maintenir le dispositif de prélèvement avec l'élément de prélèvement apparent, ce support pouvant par exemple comporter des moyens 71 permettant de rompre l'extrémité sécable alors que le dispositif de prélèvement est en place dans le support 70. Ces moyens 71 comportent par exemple une fenêtre permettant d'accéder à l'extrémité sécable ou un élément mobile par rapport au support et dont l'actionnement provoque une poussée latérale sur l'extrémité sécable. Le tube peut encore être dépourvu d'extrémité sécable mais simplement comporter une extrémité fermée et le support 70 être équipé par exemple d'une lame ou d'une pointe permettant de couper ou de percer le tube pour permettre à l'air de pénétrer à l'intérieur et au liquide et au bouchon de s'évacuer lorsque le dispositif de prélèvement est utilisé.

Bien entendu, l'invention n'est pas limitée aux exemples de réalisation qui viennent d'être décrits.

Un dispositif selon l'invention peut trouver de nombreuses applications.

L'invention peut permettre outre les applications déjà décrites, d'évaluer entre autres l'activité phosphatase acide, laquelle peut traduire le caractère plus ou moins abîmé de la peau par les détergents, la catalase, des déficits de la fonction barrière ou le pouvoir tampon de la peau.

L'extrémité du tube 21 opposée à l'extrémité par laquelle le produit sort peut par exemple être fermée autrement qu'avec une partie sécable manuellement, au moyen d'un bouchon ou d'un piston, par exemple.

Le tube 21 peut être réalisé avec une partie renflée, permettant par exemple d'exercer une pression sur le produit pour lui faire quitter le tube.

Par « tube », il faut comprendre tout corps de préférence généralement allongé, de section constante ou non, présentant au moins un canal intérieur susceptible de contenir un liquide, un tel tube pouvant présenter un axe longitudinal rectiligne ou non. L'invention n'est pas limitée à un tube de section extérieure circulaire, ni à un tube réalisé conformément à l'enseignement du brevet US 5 702 035.

Dans toute la description, y compris les revendications, l'expression « comportant un » doit être comprise comme étant synonyme de « comportant au moins un », sauf si le contraire est spécifié.

## Revendications

1. Dispositif de prélèvement (20) comportant :
- un tube (21) muni à une extrémité d'un élément de prélèvement (23),
- un bouchon (24) d'un liquide ou d'une poudre à l'intérieur du tube (21),
- au moins un produit liquide contenu dans un espace intérieur du tube séparé de l'élément de prélèvement par le bouchon au moins, ce bouchon étant agencé pour s'évacuer avec le produit liquide vers l'élément de prélèvement lors de l'utilisation, le produit liquide étant un réactif propre à produire une réaction observable en présence d'un analyte déterminé recueilli par l'élément de prélèvement (23).

2. Dispositif selon la revendication 1, **caractérisé par le fait que** l'élément de prélèvement (23) est pré-imprégné par un liquide de prélèvement.

3. Dispositif selon la revendication précédente, **caractérisé par le fait que** le liquide de prélèvement est choisi dans le groupe constitué par : le chloroforme, l'acétate d'éthyle, les alcools, les solvants chlorés, l'acétone, les esters courts, les solutions aqueuses de méthanol, les solutions de chloroforme et d'éthanol.

4. Dispositif selon l'une quelconque des revendications précédentes, **caractérisé par le fait que** l'élément de prélèvement (23) est poreux, notamment fibreux.

5. Dispositif selon l'une quelconque des revendications précédentes, **caractérisé par le fait que** l'élément de prélèvement (23) est choisi dans le groupe constitué par : un embout en coton, un embout en mousse, un embout floqué, une pointe feutre, un embout en céramique ou en un matériau fritté.

6. Dispositif selon l'une quelconque des revendications précédentes, **caractérisé par le fait que** le liquide du bouchon de liquide (24) est choisi dans le groupe constitué par : les huiles minérales, les produits fluorés, les silicones.

7. Dispositif selon l'une quelconque des revendications 1 à 5, **caractérisé par le fait que** le bouchon (24) comporte une poudre choisie dans le groupe constitué par : les poudres de microsphères de copolymères, de Nylon®, des cires, des silices et des silicones.

8. Dispositif selon l'une quelconque des revendications précédentes, **caractérisé par le fait que** l'espace intérieur du tube (21) est délimité, du côté opposé au bouchon (24) de liquide ou de poudre, par une partie sécable (22), amovible, perforable
ou déformable.

9. Dispositif selon la revendication précédente, **caractérisé par le fait qu'**il comporte un élément de maintien de la partie sécable (22) sur le dispositif de prélèvement après son sectionnement.

10. Dispositif selon l'une quelconque des revendications précédentes, **caractérisé par le fait que** le volume de liquide contenu dans le tube (21) est compris entre 0,01 ml et 5 ml, de préférence entre 0,05 ml et 1 ml.

11. Kit (20) de prélèvement et d'analyse comportant :
- au moins un dispositif de prélèvement (20) comportant :
- un tube (21) muni à une extrémité d'un élément de prélèvement (23),
- un bouchon d'un liquide ou d'une poudre (24) à l'intérieur du tube (21),
- au moins un liquide de prélèvement (L) contenu dans un espace intérieur du tube séparé de l'élément de prélèvement par au moins le bouchon de liquide
ou de poudre, le liquide de prélèvement étant propre à faciliter le prélèvement d'au moins un analyte, le bouchon (24) étant agencé pour s'évacuer avec le liquide de prélèvement (L) lors de l'utilisation,
- un réactif propre à produire une réaction observable en présence de l'analyte recueilli par l'élément de prélèvement.

12. Kit selon la revendication précédente, **caractérisé par le fait que** le liquide de prélèvement est choisi dans le groupe constitué par : le chloroforme, l'acétate d'éthyle, les alcools, les solvants chlorés, l'acétone, les esters courts, les solutions aqueuses de méthanol, les solutions de chloroforme et d'éthanol.

13. Kit selon la revendication précédente, **caractérisé par le fait qu'**il comporte un boîtier (11, 12) comportant au moins un compartiment (13) dans lequel est logé au moins un dispositif de prélèvement (20).

14. Kit selon la revendication 11, **caractérisé par le fait qu'**il comporte au moins un sachet (35,33) de conditionnement dans lequel est contenu au moins un dispositif de prélèvement.

15. Procédé pour détecter la présence et/ou la concentration d'au moins un analyte à la surface d'un tissu d'un individu, **caractérisé par le fait qu'**il comporte les étapes suivantes :
- fournir un dispositif de prélèvement (20) comportant un tube (21), un bouchon d'un liquide ou d'une poudre (24) à l'intérieur du tube, au moins un liquide de prélèvement (L) contenu dans un espace intérieur du tube délimité d'un premier côté par le bouchon de liquide ou de poudre, un élément de prélèvement à une extrémité du tube, séparé du liquide de prélèvement par le bouchon de liquide ou de poudre,
- ouvrir le tube (21) afin de permettre au liquide de prélèvement de quitter le tube, le bouchon (24) de liquide ou de poudre étant apte à s'évacuer avec le liquide de prélèvement (L),
- prélever au moins un analyte avec l'élément de prélèvement,
- mettre l'analyte en contact avec un réactif propre à produire une réaction observable en présence de cet analyte ou d'une concentration déterminée de l'analyte.

## Claims

1. An analyte-taking device (20) comprising:
a tube (21) provided at one end with an analyte-taking element (23);
. a plug (24) of a liquid or a powder inside the tube (21); and
. at least one liquid contained in an inside space of the tube separated from the analyte-taking element at least by the plug, the plug being arranged, in use, to be expelled together with the liquid towards the analyte-taking element, the liquid being a reagent suitable for producing an observable reaction in the presence of a determined analyte picked up by the analyte-taking element (23).

2. A device according to the preceding claim, **characterized by** the fact that the analyte-taking element (23) is pre-impregnated with an analyte-taking liquid.

3. A device according to the preceding claim, **characterized by** the fact that the analyte-taking liquid is selected from the group constituted by: chloroform; ethyl acetate; alcohols; chlorine-containing solvents; acetone; short esters; aqueous solutions of methanol; and solutions of chloroform and ethanol.

4. A device according to any preceding claim, **characterized by** the fact that the analyte-taking element (23) is porous, and in particular fibrous.

5. A device according to any preceding claim, **characterized by** the fact that the analyte-taking element (23) is selected from the group constituted by: a cotton bud; a foam bud; a flocked bud; a felt tip; and a tip made of ceramic or of sintered material.

6. A device according to any preceding claim, **characterized by** the fact that the liquid of the liquid plug (24) is selected from the group constituted by: mineral oils; fluorine-containing substances; and silicones.

7. A device according to any one of claims 1 to 5, **characterized by** the fact that the plug (24) comprises a powder selected from the group constituted by: powders of microspheres of copolymers, of Nylon®, of waxes, of silicas, and of silicones.

8. A device according to any preceding claim, **characterized by** the fact that the inside space of the tube (21) is defined, at its end remote from the liquid or powder plug (24) by a portion (22) that can be broken off, removed, perforated, or deformed.

9. A device according to the preceding claim, **characterized by** the fact that it includes a retaining element for retaining the break-off portion (22) on the analyte-taking device after it has been broken off.

10. A device according to any preceding claim, **characterized by** the fact that the volume of liquid contained in the tube (21) lies in the range 0.01 ml to 5 ml, and preferably in the range 0.05 ml to 1 ml.

11. A kit for taking and analyzing an analyte (20), the kit comprising:
· at least one analyte-taking device (20) comprising:
. a tube (21) provided at one end with an analyte-taking element (23);
· a plug (24) of a liquid or a powder inside the tube (21);
. at least one analyte-taking liquid (L) contained in an inside space of the tube separated from the analyte-taking element at least by the liquid or powder plug, the analyte-taking liquid being suitable for facilitating the taking of at least one analyte, the plug (24) being arranged in use to be expelled together with the analyte-taking liquid (L); and
. a reagent suitable for producing an observable reaction in the presence of the analyte picked up by the analyte-taking element.

12. A kit according to the preceding claim, **characterized by** the fact that the analyte-taking liquid is selected from the group constituted by : chloroforme; ethyl acetate; alcohols; chlorine-containing solvents; acetone; short esters; aqueous solutions of methanol; and solutions of chloroform and ethanol.

13. A kit according to the preceding claim, **characterized by** the fact that it comprises a box (11, 12) including at least one compartment (13) in which at least one analyte-taking device (20) is housed.

14. A kit according to claim 11, **characterized by** the fact that it comprises at least one packaging bag (35, 33) containing at least one analyte-taking device.

15. A method of detecting the presence and/or the concentration of at least one analyte at the surface of a tissue of an individual, the method being **characterized by** the fact that it comprises the following steps:
· providing an analyte-taking device (20) comprising a tube (21), a plug (24) of a liquid or a powder inside a tube, at least one analyte-taking liquid (L) contained in an inside space of the tube defined at a first end by the liquid or powder plug, and an analyte-taking element at one end of the tube, separated from the analyte-taking liquid by the liquid or powder plug;
· opening the tube (21) so as to allow the analyte-taking liquid to leave the tube, the liquid or powder plug (24) being suitable for being expelled together with the analyte-taking liquid (L);
. taking at least one analyte with the analyte-taking element; and
· putting the analyte into contact with a reagent suitable for producing an observable reaction in the presence of said analyte or in the presence of a determined concentration of the analyte.

## Patentansprüche

1. Entnahmevorrichtung (20), umfassend:
- ein Rohr (21), das an einem Ende mit einem Entnahmeelement (23) versehen ist,
- einen Stopfen (24) aus einer Flüssigkeit oder einem Pulver im Inneren des Rohrs (21),
- mindestens ein flüssiges Produkt, das in einem Innenraum des Rohrs enthalten ist, der von dem Entnahmeelement mindestens durch den Stopfen getrennt ist, wobei dieser Stopfen ausgebildet ist, um bei der Verwendung mit dem flüssigen Produkt auf das Entnahmeelement zu abgeführt zu werden, wobei das flüssige Produkt ein Reagens ist, das geeignet ist, in Gegenwart eines bestimmten, von dem Entnahmeelement (23) aufgenommenen Analyten eine beobachtbare Reaktion zu erzeugen.

2. Vorrichtung nach Anspruch 1, **dadurch gekennzeichnet, dass** das Entnahmeelement (23) mit einer Entnahmeflüssigkeit vorgetränkt ist.

3. Vorrichtung nach dem vorhergehenden Anspruch, **dadurch gekennzeichnet, dass** die Entnahmeflüssigkeit in der Gruppe gewählt ist, die besteht aus: Chloroform, Ethylacetat, Alkoholen, Chlorlösungsmitteln, Aceton, kurzen Estern, wässrigen Methanollösungen, Chloroform- und Ethanollösungen.

4. Vorrichtung nach einem der vorhergehenden Ansprüche, **dadurch gekennzeichnet, dass** das Entnahmeelement (23) porös, insbesondere fasrig ist.

5. Vorrichtung nach einem der vorhergehenden Ansprüche, **dadurch gekennzeichnet, dass** das Entnahmeelement (23) in der Gruppe gewählt ist, die besteht aus: einem Baumwollansatz, einem Schaumansatz, einem beflockten Ansatz, einer Filzspitze, einem Ansatz aus Keramik oder einem Sintermaterial.

6. Vorrichtung nach einem der vorhergehenden Ansprüche, **dadurch gekennzeichnet, dass** die Flüssigkeit des Flüssigkeitsstopfens (24) in der Gruppe gewählt ist, die besteht aus: Mineralölen, Fluorprodukten, Siliconen.

7. Vorrichtung nach einem der Ansprüche 1 bis 5, **dadurch gekennzeichnet, dass** der Stopfen (24) ein Pulver umfasst, das in der Gruppe gewählt ist, die besteht aus: Pulvern von Mikrokugeln aus Copolymeren, Nylon®, Wachsen, Siliciumoxiden und Siliconen.

8. Vorrichtung nach einem der vorhergehenden Ansprüche, **dadurch gekennzeichnet, dass** der Innenraum des Rohrs (21) auf der dem Stopfen (24) aus Flüssigkeit oder Pulver entgegengesetzten Seite durch einen durchtrennbaren, abnehmbaren, perforierbaren oder verformbaren Teil (22) begrenzt ist.

9. Vorrichtung nach dem vorhergehenden Anspruch, **dadurch gekennzeichnet, dass** sie ein Element zum Halten des durchtrennbaren Teils (22) an der Entnahmevorrichtung nach seinem Abtrennen umfasst.

10. Vorrichtung nach einem der vorhergehenden Ansprüche, **dadurch gekennzeichnet, dass** das in dem Rohr (21) enthaltene Flüssigkeitsvolumen zwischen 0,01 ml und 5 ml, vorzugsweise zwischen 0,05 ml und 1 ml, beträgt.

11. Entnahme- und Analyseset (20), umfassend:
- mindestens eine Entnahmevorrichtung (20), umfassend:
- ein an einem Ende mit einem Entnahmeelement (23) versehenes Rohr (21),
- einen Stopfen (24) aus einer Flüssigkeit oder einem Pulver im Inneren des Rohrs (21),
- mindestens eine Entnahmeflüssigkeit (L), die in einem Innenraum des Rohrs enthalten ist, der von dem Entnahmeelement mindestens durch den Flüssigkeits- oder Pulverstopfen getrennt ist, wobei die Entnahmeflüssigkeit geeignet ist, die Entnahme mindestens eines Analyten zu erleichtern, wobei der Stopfen (24) dafür ausgelegt ist, bei der Verwendung mit der Entnahmeflüssigkeit (L) abgeführt zu werden,
- ein Reagens, das geeignet ist, in Gegenwart des von dem Entnahmeelement aufgenommenen Analyten eine beobachtbare Reaktion zu erzeugen.

12. Set nach dem vorhergehenden Anspruch, **dadurch gekennzeichnet, dass** die Entnahmeflüssigkeit in der Gruppe ausgewählt ist, die besteht aus: Chloroform, Ethylacetat, Alkoholen, Chlorlösungsmitteln, Aceton, kurzen Estern, wässrigen Methanollösungen, Chloroform- und Ethanollösungen.

13. Set nach dem vorhergehenden Anspruch, **dadurch gekennzeichnet, dass** es ein Gehäuse (11, 12) umfasst, das mindestens ein Abteil (13) umfasst, in dem mindestens eine Entnahmevorrichtung (20) untergebracht ist.

14. Set nach Anspruch 11, **dadurch gekennzeichnet, dass** es mindestens einen Verpackungsbeutel (35, 33) umfasst, in dem mindestens eine Entnahmevorrichtung enthalten ist.

15. Verfahren zum Erfassen des Vorhandenseins und/oder der Konzentration mindestens eines Analyten auf der Oberfläche eines Gewebes einer Person, **dadurch gekennzeichnet, dass** es die folgenden Schritte umfasst:
- eine Entnahmevorrichtung (20) liefern, umfassend ein Rohr (21), einen Stopfen (24) aus einer Flüssigkeit oder einem Pulver im Inneren des Rohrs, mindestens eine Entnahmeflüssigkeit (L), die in einem Innenraum des Rohrs enthalten ist, der auf einer ersten Seite durch den Flüssigkeits-oder Pulverstopfen begrenzt ist, ein Entnahmeelement an einem Ende des Rohrs, das von der Entnahmeflüssigkeit durch den Flüssigkeits- oder Pulverstopfen getrennt ist,
- das Rohr (21) öffnen, um der Entnahmeflüssigkeit zu gestatten, das Rohr zu verlassen, wobei der Flüssigkeits-oder Pulverstopfen (24) mit der Entnahmeflüssigkeit (L) abgeführt werden kann,
- mindestens einen Analyten mit dem Entnahmeelement entnehmen,
- den Analyten mit einem Reagens in Kontakt bringen, das geeignet ist, in Gegenwart dieses Analyten oder einer bestimmten Konzentration des Analyten eine beobachtbare Reaktion zu erzeugen.
